# EUROPEAN PATENT SPECIFICATION

(11) **EP 3 281 583 B1**
(45) Date of publication and mention of the grant of the patent: **08.05.2019**
(21) Application number: 17160388.9
(22) Date of filing: 10.03.2017
(51) Int. Cl.: A61B 5/05

(54) **A MEDICAL IMAGING SYSTEM AND METHOD**
SYSTEM UND VERFAHREN ZUR MEDIZINISCHEN BILDGEBUNG
SYSTÈME D'IMAGERIE MÉDICALE ET PROCÉDÉ ASSOCIÉ

(30) Priority: 12.08.2016 GB 201613879
(43) Date of publication of application: 14.02.2018
(73) Proprietor: Micrima Limited, Bristol BS2 0EL (GB)
(72) Inventor: BORE, Chris Frank, Bristol, BS2 0EL (GB)
(74) Representative: Haseltine Lake LLP

(56) References cited:
- WO-A1-2012/048020
- KR-A- 20160 040 510
- US-A- 5 713 356
- US-A1- 2009 015 832
- SOLLIP KWON ET AL: "Recent Advances in Microwave Imaging for Breast Cancer Detection", INTERNATIONAL JOURNAL OF BIOMEDICAL IMAGING, vol. 2016, 1 January 2016 (2016-01-01), pages 1-25, XP055364408, ISSN: 1687-4188, DOI: 10.1155/2016/5054912

## Description

The invention relates to a medical imaging system and method.

Various medical imaging techniques are known for examining the human body. Such imaging techniques may be used to interrogate tissues and organs in order to identify abnormalities, such as tumours or lesions. For example, X-ray (mammography), microwave imaging, ultrasound, MRI are all common imaging modalities. Such techniques are commonly used to examine breast tissue, but may also be used in other areas of the body, such as the liver, pancreas, prostate, thyroid, lungs, ovaries and lymph nodes.

Various imaging techniques and associated technologies are described in US 2009/015832, US 5713356, Sollip Kwon ET AL: "Recent Advances in Microwave Imaging for Breast Cancer Detection", International Journal of Biomedical Imaging, vol. 2016, 1 January 2016 (2016-01-01), pages 1-25, XP055364408, ISSN: 1687-4188, DOI: 10.1155/2016/5054912, KR 10-2016-0040510 and WO 2012/048020.

The quality of the image produced by an imaging modality is a key factor in its uptake. In particular, the spatial resolution and presence or absence of artefacts in the image are fundamental properties which may be considered when selecting an imaging modality. As a result, there is generally a desire to improve the quality of the image produced using an imaging modality.

The invention seeks to provide a system which improves the quality of the image obtained using a microwave antenna array.

The invention is defined in the attached independent claims to which reference should now be made. Further, optional features are defined in the sub-claims appended thereto.

In accordance with an aspect of the invention, there is provided a medical imaging system as claimed in claim 1.

The microwave antenna array may be formed on a substrate, wherein the substrate is contoured to conform to the body part.

The substrate may be hemispherical.

The actuator may be configured to rotate the microwave antenna array relative to the body part.

The physical spacing between the antennae may be greater than half the wavelength of the microwave signal and an effective spacing between the antennae at the positions defined by the plurality of configurations may be less than half the wavelength of the microwave signal.

The antennae may be positioned in a formation formed from a regular tessellation of a polygon which is tilted with respect to a direction of movement of the antennae.

The polygon may be a square.

The formation may be tilted at an angle which is between 20 degrees and 35 degrees, and preferably at an angle of 27 degrees or more specifically 26.565 degrees in order to maximise the spacing between the antennae. In particular, with this arrangement the antennae are spaced at uniform spacings aligned parallel to the axis of rotation.

In accordance with an aspect of the invention, there is provided a medical imaging method as claimed in claim 9.

The antennae may be moved using an actuator connected to the microwave antenna array.

The actuator may be configured to rotate the microwave antenna array relative to the body part.

For a better understanding of the invention, and to show more clearly how it may be carried into effect, reference will now be made, by way of example, to the accompanying drawings, in which:-
Figure 1 is a system diagram of a medical imaging system according to an embodiment of the invention;
Figure 2 is a schematic view of the medical imaging system;
Figure 3 is a flowchart depicting a sampling method;
Figure 4 is a plan view of an antenna array of the medical imaging system in a first configuration;
Figure 5 is a plan view of the antenna array in a second configuration;
Figure 6 is a plan view of the antenna array in a third configuration;
Figure 7 shows the positions of the antennae for each configuration shown in Figures 4 to 6 overlaid on one another; and
Figure 8 is an illustrative example showing a translation of a tilted tessellation.

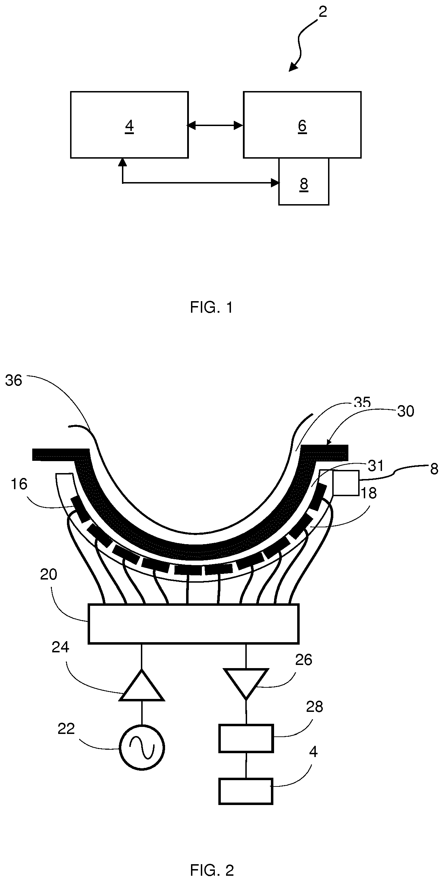

Figure 1 shows a medical imaging system 2 according to an embodiment of the invention. The medical imaging system generally comprises a processor 4, a microwave antenna array 6 in communication with the processor 4, and an actuator 8 connected to the microwave antenna array 6 and in communication with the processor 4.

As shown in Figure 2, the antenna array 6 comprises a plurality N of antennae 16 which are arranged over the surface of, or within, a shell substrate 18. The shell 18 has a curved profile as shown. In particular, the shell 18 is part or hemi-spherical and is configured to approximate the shape of a breast. The antennae 16 are arranged over the shell 18 such that they all point to a common focal point.

The antennae 16 are each electrically connected to a switching matrix 20. The switching matrix 20 is in turn connected to both a transmit path and a receive path. The transmit path comprises a signal generator 22 coupled to an amplifier 24. The receive path comprises an amplifier 26 coupled to a detector 28 and the processor 4.

The switching matrix 20 selectively couples each of the antennae 16 to either the transmit path or the receive path.

The antenna array 6 is operated in a multi-static fashion. Specifically, the switching matrix 20 is controlled so as to connect one of the antennae 16 to the transmit path and the remaining antennae 16 to the receive path. The signal generator 22 generates a stepped frequency continuous wave (CW) signal which is amplified by the amplifier 24 and then transmitted by the antenna 16 connected to the transmit path. The stepped frequency continuous wave signal is a sequential series of pulses of continuous wave energy, where each pulse has its frequency stepped up across a range of frequencies, typically within the 3-8 GHz range. The other antennae 16 receive the transmitted signal and the received signal is detected and then recorded by the processor 4.

As shown in Figure 2, the shell 18 receives a cup 30. The cup 30 has a complementary shape to the shell 18 such that if fits snugly within the shell 18. A layer of coupling fluid (dielectric constant controlled fluid) may be inserted in the gap 31 between the shell 18 and the cup 30 so as to improve the coupling between the antennae 16 and the cup 30 in order to minimise signal loss and thus improve transmission of the microwave signal.

As described previously, an actuator 8, such as a motor, is connected to the microwave antenna array 6. The actuator 8 is configured to move the microwave antenna array 6 relative to the cup 30 which remains stationary against the breast 36. Specifically, the actuator 8 causes the microwave antenna array 6 to rotate relative to the cup 30 about the breast 36. The microwave antenna array 6 rotates about the center of the shell 18 (i.e. its axis of symmetry). This may be enabled by a threaded engagement between the cup 30 and the shell 18. In particular, the outside of the cup 30 and the inside of the shell 18 may have threaded portions which engage to allow the shell 18 and the antenna array 6 to be rotated relative to the cup 30. This may also allow the cup 30 to be quickly and easily removed so as to enable the coupling fluid to be replaced.

The antennae 16 may be as described in WO 2009/060181. In particular, the antennae 16 may comprise a slot 16 formed in a conductive element, the slot having a rectangular external boundary defined by a substantially closed internal edge of the conductive element. A microstrip feed line may be spaced from the conductive element by a dielectric substrate with the distal end of the line positioned at the geometric centre of the slot. Although such an antenna is effective at launching microwaves into human tissue, its size may prevent the antennae from being positioned sufficiently close to one another. The system 2 uses the actuator 8 to rotate the antenna array 6 so that the antennae 16 assume positions which were previously unoccupied. Although the positions of the antennae 16 may overlap to a certain extent, the term "unoccupied" is used herein to refer to new sampling positions. The rotation of the antenna array 6 therefore creates a "virtual" array which has a higher density of antennae 16.

In use, a patient lies in a prone position such that their breast 36 sits in the cup 30. A layer of coupling fluid may also be provided in the gap 35 between the cup 30 and the breast 36 in order to improve coupling between the antennae 16 and the breast 36.

Although not shown, one or more inserts may be placed inside the cup 30 so as to enable a better fit between the internal surface of the cup 30 and the breast 36. For example, a plurality of such inserts may be provided, each having different shapes and sizes, to enable the system to be better adapted to breasts of different shapes and sizes. The inserts may be made from the same material as the cup (e.g. ceramic).

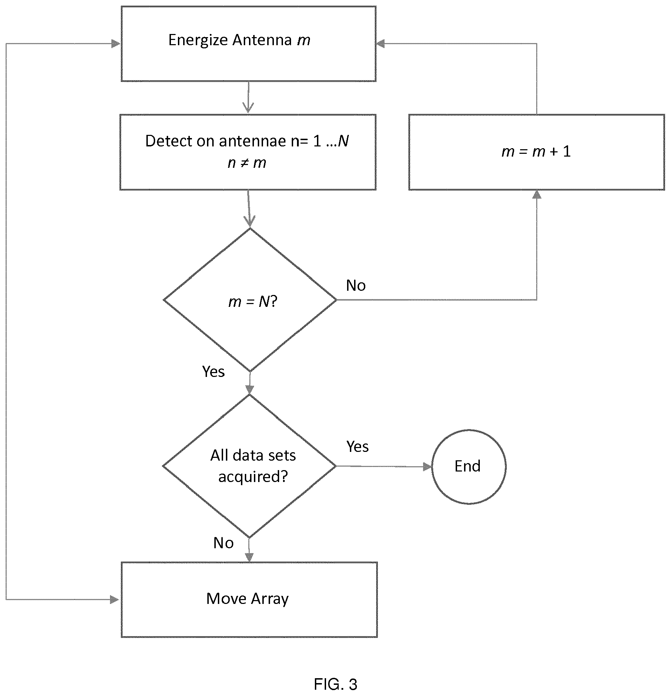

Figure 3 shows a flowchart of a data acquisition method. As shown, the switching matrix 20 connects an antenna *m* of the *N* antennae to the transmit path. All other antennae 16 (*n* = *1...N, n*≠*m*) are connected to the receive path. The antenna 16 connected to the transmit path illuminates the breast 36 with the microwave signal. The signal is scattered by the breast tissue and the scattered signal is received at each of the non-transmitting antennae 16 where it is detected (possibly in a time-sharing arrangement) and recorded. If *m*≠*N*, the switching matrix 20 steps to the next antenna 16 (*m* = *m* + *1*) to be connected to the transmit path. This is repeated until all antennae 16 have been connected to the transmit path.

If additional data sets are required, then the actuator 8 is used to rotate the array 6 relative to the breast 36 while retaining the breast in position. The acquisition process is then repeated with the antenna array 6 in the new configuration.

The processor 4 may record the relative difference between the measured phase and amplitude of the transmitted signal as compared to the phase and amplitude of the scattered signal, recorded as a complex number (having real and imaginary parts).

The signal detected at each antenna 16 is affected by scattering arising from objects within the imaged volume (i.e. the breast 36). In particular, tumours can generate significant reflections as they exhibit much higher dielectric properties than adipose and connective tissues due to their significant water content and so can be identified in the acquired data.

The acquired data sets may be used by the processor 4 to construct an image of the internal structure of the breast 36. Specifically, the data sets acquired for each configuration of the antenna array 6 are concatenated (i.e. combined) and the concatenated data set is used to generate the image. The resolution of the image is therefore improved by combining the data sets for each configuration of the antenna array 6. The Data reconstruction may be performed using Phased Array (frequency domain), Delay and Sum (DAS - time domain) techniques or any other suitable technique, such as 3D Fourier Transformation, Back Projection, etc. From this, the processor 4 is able to identify (possibly, with additional user input or confirmation) a region of interest (if present) in which a possible tumour or other pathology may exist.

As described above, the rotation of the antenna array 6 relative to the breast 36 during the image capture process moves the antennae 16 to previously unoccupied positions. The effective spacing of the antennae 16 (i.e. the spacing between the occupied positions across all configurations) in the virtual array is therefore smaller than the physical spacing of the antennae 16 on the shell 18. This may therefore improve the resolution of the image. The effective spacing of the antennae 16 in the virtual array is preferably less than half the wavelength of the microwave signal in order to prevent grating lobes from occurring in the image. Owing to the size of the antennae 16, such spacing may not otherwise be physically achievable. As described previously, the frequency of the microwave signal may be stepped up across a range of frequencies, typically within the 3-8 GHz range. Accordingly, the wavelength referred to here is based on the centre frequency, i.e. 5.5 GHz (which at εᵣ≅9, λ≅2cm).

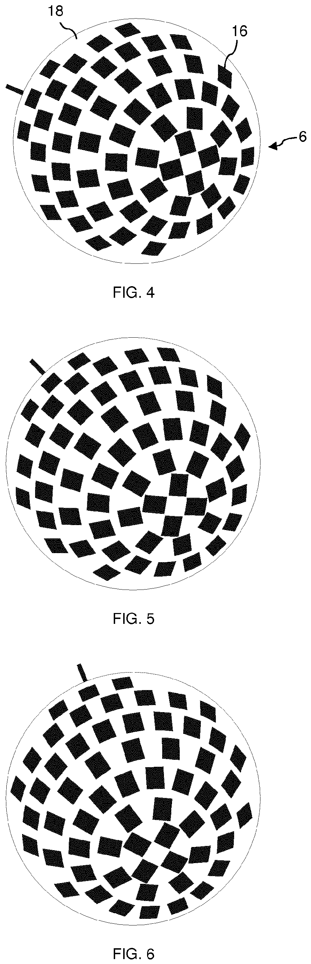

Although the antennae 16 may be positioned over the shell 18 in any arrangement which causes them to be moved to previously unoccupied positions, Figures 4 to 6 show a preferred arrangement which maximises coverage. Figure 4 shows the antenna array 6 in a first configuration and Figures 5 and 6 show the antenna array 6 in second and third configurations where the array has been rotated about an axis of rotation which is preferably aligned with the axis of symmetry of the antenna array 6.

The arrangement shown in Figures 4 to 6 is based on a projection into a hemispherical form of a tilted regular tessellation (tiling) of polygons, where the corners of the tiles define the positions of the antennae 16.

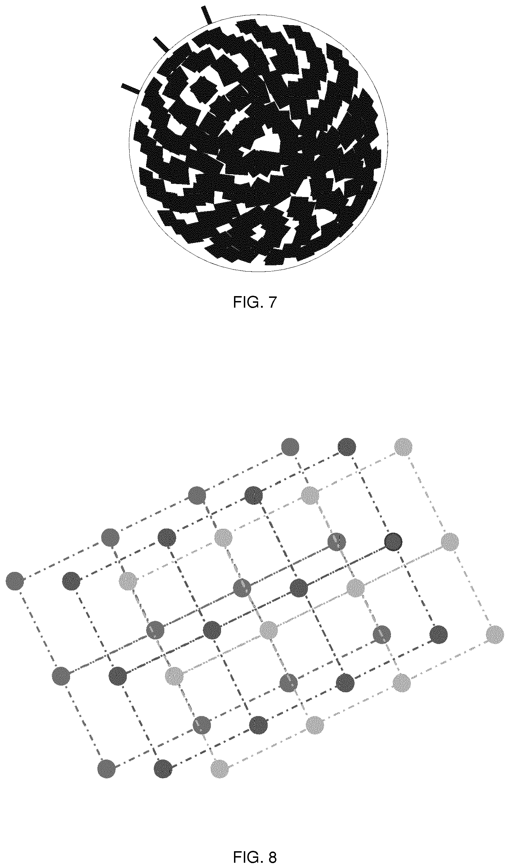

For a simple tessellation of unit squares, a horizontal translation by 0.5 will generate a virtual array which has a reduced effective spacing in the horizontal direction, but which retains the same vertical spacing. In contrast, a diagonal translation (movement along two axes of motion - vertical and horizontal) will reduce the effective spacing in both the horizontal and vertical directions. This same effect can be achieved by instead tilting the tessellation of squares and then performing a translation along a single axis (e.g. horizontal), as shown in Figure 8.

By tilting the tessellation the corners of the squares move along paths which are all offset from one another, rather than moving along common paths. A uniform vertical spacing between the corners may be achieved by tilting the square tessellation by 26.565 degrees (=tan⁻¹(0.5)). This maximises the vertical separation between the corners.

As described above, the arrangement shown in Figures 4 to 6 is a projection of the tilted tessellation into a hemispherical form. The tilted tessellation may be transformed into a hemispherical form using an inverse Mercator projection or other map projection. Rotation of the antenna array 6 about its axis thus causes the antennae 16 to occupy new positions which are spaced between the original positions of the antennae 16 so as to define a virtual array which has a substantially uniform density with an effective spacing which satisfies the half wavelength criterion. Figure 7 shows the positions of the antennae 16 for each configuration overlaid on one another and thus demonstrates the density of the virtual array.

Typically in a hemispherical array where the antenna are separated by a single angular distance A, the angular rotation for each increment will be A/3, thereby increasing the virtual array density by a factor of three.

The density of the positions shown in Figure 7 is sufficient to produce a concatenated data set which is completely determined. In other words, from the concatenated data set, the interference pattern that is the scattered field may be calculated at any spatial position, including at positions other than those of the samples. Thus from a set of discrete samples complete information may be obtained about the field at all spatial positions. This avoids artefacts such as grating lobes from occurring within the image. The movement of the antennae 16 reduces the effective spacing between the antennae 16. This therefore allows larger and/or fewer antennae to be used while still obtaining an effective spacing which is less than half the microwave wavelength.

Although the antenna array 6 has been described as having a plurality of discrete configurations/positions, the data may instead be acquired from a continuously moving array. Further, the entire antenna array 6 need not rotate and the individual antennae 16 may instead assume different positions within the shell 18 in each configuration of the antenna array 6.

Although the system has been described with reference to the imaging of a breast, it will be appreciated that it may be adapted for other areas of the body. The antenna array 6 therefore need not be hemispherical and may be contoured to conform to other body parts. Where the antenna array 6 does not have rotational symmetry, the antennae 16 may be translated rather than rotated.

The actuator 8 may cause movement of the antennae 16 in any suitable way. In other arrangements, the movement of the antennae 16 may be manually actuated. Where manual actuation is used, the antenna array 6 may be arranged so as to ensure repeatability of the measurements. In particular, the antenna array 6 may have discrete positions and a suitable form of feedback for confirming to the user when the antenna array 6 is in the proper position. The antenna array 6 may be biased towards discrete positions using a suitable indexing mechanism or the like.

It will be appreciated that the processor 4 need not display, or indeed, generate, an image of the internal structure of the breast in order to identify a region of interest. The region of interest may instead be determined based on the raw data.

The system may employ multiple transmit and receive paths, such that data can be recorded from multiple antennas simultaneously, and may even comprise a number of transmit and receive paths corresponding to the number of antennas, such that data can be recorded from all antennas simultaneously. In an alternative topology, the switching matrix could be removed thus allowing each antenna to be connected to a transmitting/receiving device.

To avoid unnecessary duplication of effort and repetition of text in the specification, certain features are described in relation to only one or several aspects or embodiments of the invention. However, it is to be understood that, where it is technically possible, features described in relation to any aspect or embodiment of the invention may also be used with any other aspect or embodiment of the invention.

The invention is not limited to the embodiments described herein, and may be modified or adapted without departing from the scope of the present invention, as defined by the claims.

## Claims

1. A medical imaging system (2) comprising:
a microwave antenna array (6) comprising a plurality of antennae (16) which are spaced from one another, the plurality of antennae (16) defining a transmitting antenna and receiving antennae (16), wherein the transmitting antenna is configured to transmit microwave signals so as to illuminate a body part of a patient and the receiving antennae (16) are configured to receive the microwave signals following scattering within the body part;
wherein the microwave antenna array (6) has a plurality of configurations defining positions of the plurality of antennae (16) relative to the body part, wherein in each configuration the plurality of antennae (16) are located at positions which are unoccupied in the other configurations;
the medical imaging system (2) further comprising:
an actuator (8) configured to move the microwave antenna array (6) between the plurality of configurations so as to position the plurality of antennae (16) at previously unoccupied positions; and
a processor (4) configured to obtain a data set for the microwave signals produced in each of the plurality of configurations of the microwave antenna array (6) and to generate an output indicative of the internal structure of the body part from a concatenation of the data sets; **characterized in that**:
the physical spacing between the antennae (16) is greater than half the wavelength of the microwave signal and an effective spacing between the antennae (16), defined as the spacing between the occupied positions across all configurations, is less than half the wavelength of the microwave signal; and **in that**
the antennae (16) are positioned in a formation formed from a regular tessellation of a polygon which is tilted with respect to a direction of movement of the antennae (16).

2. A medical imaging system (2) as claimed in claim 1, wherein the microwave antenna array (6) is formed on a substrate, wherein the substrate is contoured or contourable to conform to the body part.

3. A medical imaging system (2) as claimed in claim 2, wherein the substrate is hemispherical.

4. A medical imaging system (2) as claimed in any preceding claim, wherein the actuator (8) is configured to rotate the microwave antenna array (6) relative to the body part.

5. A medical imaging system (2) as claimed in any preceding claim, wherein the antennae (16) are positioned in a formation which is formed from an inverse Mercator projection of the regular tessellation.

6. A medical imaging system (2) as claimed in any preceding claim, wherein the polygon is a square.

7. A medical imaging system (2) as claimed in any preceding claim, wherein the formation is tilted at an angle which is between 20 degrees and 35 degrees.

8. A medical imaging system (2) as claimed in claim 7 the formation is tilted at an angle of 27 degrees.

9. A medical imaging method comprising:
(a) illuminating a body part of a patient with microwave signals emitted by a transmitting antenna of a microwave antenna array (6);
(b) receiving the microwave signals following scattering within the body part at a plurality of receiving antennae (16) of the microwave antenna array (6) to obtain a data set;
(c) moving the positions of the antennae (16) relative to the body part so that they are located at positions which were previously unoccupied and repeating steps (a) and (b);
(d) concatenating the data sets obtained and generating an output indicative of the internal structure of the body part from the concatenated data sets; **characterized in that**:
the physical spacing between the antennae (16) is greater than half the wavelength of the microwave signal and an effective spacing between the antennae (16), defined as the spacing between occupied positions across all configurations and achieved by moving the antennae (16) to previously unoccupied positions is less than half the wavelength of the microwave signal; and **in that**
the antennae (16) are positioned in a formation formed from a regular tessellation of a polygon which is tilted with respect to a direction of movement of the antennae (16).

10. A medical imaging method as claimed in claim 9, wherein the antennae (16) are moved using an actuator (8) connected to the microwave antenna array (6).

11. A medical imaging method as claimed in claim 10, wherein the actuator (8) is configured to rotate the microwave antenna array (6) relative to the body part.

## Patentansprüche

1. System (2) zur medizinischen Bildgebung, umfassend:
ein Mikrowellenantennenarray (6) umfassend mehrere Antennen (16), die voneinander beabstandet sind, wobei die mehreren Antennen (16) eine Sendeantenne und eine Empfangsantenne (16) definieren, wobei die Sendeantenne ausgebildet ist zum Übertragen von Mikrowellensignalen, um ein Körperteil eines Patienten zu beleuchten, und die Empfangsantennen (16) ausgebildet sind zum Empfangen der Mikrowellensignale nach dem Streuen innerhalb des Körperteils;
wobei das Mikrowellenantennenarray (6) mehrere Ausbildungen besitzt, die Positionen der mehreren Antennen (16) relativ zu dem Körperteil definieren, wobei in jeder Ausbildung sich die mehreren Antennen (16) in Positionen befinden, die in den anderen Ausbildungen unbesetzt sind;
wobei das System (2) zur medizinischen Bildgebung weiterhin umfasst:
einen Aktuator (8), der ausgebildet ist zum Bewegen des Mikrowellenantennenarrays (6) zwischen den mehreren Ausbildungen, um die mehreren Antennen (16) an zuvor unbesetzten Positionen zu positionieren; und
einen Prozessor (4), der ausgebildet ist zum Erhalten eines Datensatzes für die in jeder der mehreren Ausbildungen des Mikrowellenantennenarrays (6) erzeugten Mikrowellensignale und zum Generieren einer Ausgabe, die die interne Struktur des Körperteils anzeigt, anhand einer Verknüpfung der Datensätze; **dadurch gekennzeichnet, dass**:
der physische Abstand zwischen den Antennen (16) größer ist als die Hälfte der Wellenlänge des Mikrowellensignals, und ein effektiver Abstand zwischen der Antenne (16), definiert als der Abstand zwischen den besetzten Positionen über alle Ausbildungen, kleiner ist als die Hälfte der Wellenlänge des Mikrowellensignals; und dass
die Antennen (16) in einer Formation positioniert sind, die aus einer regelmäßigen Tesselierung eines Polygons ausgebildet wird, das bezüglich einer Bewegungsrichtung der Antennen (16) geneigt ist.

2. System (2) zur medizinischen Bildgebung nach Anspruch 1, wobei das Mikrowellenantennenarray (6) auf einem Substrat ausgebildet ist, wobei das Substrat kontouriert oder kontourierbar ist, um konform zu dem Körperteil zu sein.

3. System (2) zur medizinischen Bildgebung nach Anspruch 2, wobei das Substrat halbkugelförmig ist.

4. System (2) zur medizinischen Bildgebung nach einem vorhergehenden Anspruch, wobei der Aktuator (8) ausgebildet ist zum Drehen des Mikrowellenantennenarrays (6) relativ zum Körperteil.

5. System (2) zur medizinischen Bildgebung nach einem vorhergehenden Anspruch, wobei die Antennen (16) in einer Formation positioniert sind, die aus einer inversen Mercatorprojektion der regelmäßigen Tesselierung ausgebildet ist.

6. System (2) zur medizinischen Bildgebung nach einem vorhergehenden Anspruch, wobei das Polygon ein Quadrat ist.

7. System (2) zur medizinischen Bildgebung nach einem vorhergehenden Anspruch, wobei die Formation unter einem Winkel geneigt ist, der zwischen 20 Grad und 35 Grad liegt.

8. System (2) zur medizinischen Bildgebung nach Anspruch 7, wobei die Formation unter einem Winkel von 27 Grad geneigt ist.

9. Verfahren zur medizinischen Bildgebung, umfassend:
(a) Beleuchten eines Körperteils eines Patienten mit durch eine Sendeantenne eines Mikrowellenantennenarrays (6) emittierten Mikrowellensignalen;
(b) Empfangen der Mikrowellensignale nach dem Streuen innerhalb des Körperteils an mehreren Empfangsantennen (16) des Mikrowellenantennenarrays (6), um einen Datensatz zu erhalten;
(c) Bewegen der Positionen der Antennen (16) relativ zum Körperteil, so dass sie sich an Positionen befinden, die zuvor unbesetzt waren, und Wiederholen der Schritte (a) und (b);
(d) Verknüpfen der erhaltenen Datensätze und Generieren einer Ausgabe, die die interne Struktur des Körperteils anzeigt, aus den verknüpften Datensätzen; **dadurch gekennzeichnet, dass**:
der physische Abstand zwischen den Antennen (16) größer ist als die Hälfte der Wellenlänge des Mikrowellensignals, und ein effektiver Abstand zwischen den Antennen (16), definiert als der Abstand zwischen besetzten Positionen über alle Ausbildungen und durch Bewegen der Antenne (16) zu zuvor unbesetzten Positionen erzielt, kleiner ist als die Hälfte der Wellenlänge des Mikrowellensignals; und dass
die Antennen (16) in einer Formation positioniert sind, die aus einer regelmäßigen Tesselierung eines Polygons ausgebildet wird, das bezüglich einer Bewegungsrichtung der Antennen (16) geneigt ist.

10. Verfahren zur medizinischen Bildgebung nach Anspruch 9, wobei die Antennen (16) unter Verwendung eines mit dem Mikrowellenantennenarray (6) verbundenen Aktuators (8) bewegt werden.

11. Verfahren zur medizinischen Bildgebung nach Anspruch 10, wobei der Aktuator (8) ausgebildet ist zum Drehen des Mikrowellenantennenarrays (6) relativ zum Körperteil.

## Revendications

1. Système (2) d'imagerie médicale comprenant :
un réseau (6) d'antennes micro-ondes comprenant de multiples antennes (16) qui sont espacées les unes des autres, les multiples antennes (16) définissant une antenne émettrice et des antennes réceptrices (16), dans lequel l'antenne émettrice est conçue pour émettre des signaux micro-ondes de façon à éclairer une partie corporelle d'un patient et les antennes réceptrices (16) sont conçues pour recevoir les signaux micro-ondes après diffusion dans la partie corporelle ;
dans lequel le réseau (6) d'antennes micro-ondes possède de multiples configurations définissant des positions des multiples antennes (16) par rapport à la partie corporelle, dans lequel, dans chaque configuration, les multiples antennes (16) sont situées à des positions qui sont inoccupées dans les autres configurations ;
le système (2) d'imagerie médicale comprenant en outre :
un actionneur (8) conçu pour déplacer le réseau (6) d'antennes micro-ondes entre les multiples configurations de façon à positionner les multiples antennes (16) à des positions précédemment inoccupées ; et
un processeur (4) configuré pour obtenir un ensemble de données pour les signaux micro-ondes produits dans chaque configuration de la pluralité de configurations du réseau (6) d'antennes micro-ondes et pour générer une sortie indiquant la structure interne de la partie corporelle à partir d'une concaténation des ensembles de données ; **caractérisé en ce que** :
l'espacement physique entre les antennes (16) est supérieur à la moitié de la longueur d'onde du signal micro-ondes et un espacement efficace entre les antennes (16), défini comme étant l'espacement entre les positions occupées sur toutes les configurations, est inférieur à la moitié de la longueur d'onde du signal micro-ondes ; et **en ce que**
les antennes (16) sont positionnées dans une formation formée à partir d'une tessellation normale d'un polygone qui est incliné par rapport à une direction de déplacement des antennes (16).

2. Système (2) d'imagerie médicale selon la revendication 1, dans lequel le réseau (6) d'antennes micro-ondes est formé sur un substrat, le substrat étant profilé ou pouvant l'être pour épouser la forme de la partie corporelle.

3. Système (2) d'imagerie médicale selon la revendication 2, dans lequel le substrat est hémisphérique.

4. Système (2) d'imagerie médicale selon l'une quelconque des revendications précédentes, dans lequel l'actionneur (8) est conçu pour faire tourner le réseau (6) d'antennes micro-ondes par rapport à la partie corporelle.

5. Système (2) d'imagerie médicale selon l'une quelconque des revendications précédentes, dans lequel les antennes (16) sont positionnées dans une formation qui est formée à partir d'une projection de Mercator inversée de la tessellation normale.

6. Système (2) d'imagerie médicale selon l'une quelconque des revendications précédentes, dans lequel le polygone est un carré.

7. Système (2) d'imagerie médicale selon l'une quelconque des revendications précédentes, dans lequel la formation est inclinée selon un angle qui est entre 20 degrés et 35 degrés.

8. Système (2) d'imagerie médicale selon la revendication 7, la formation étant inclinée selon un angle de 27 degrés.

9. Procédé d'imagerie médicale consistant à :
(a) éclairer une partie corporelle d'un patient avec des signaux micro-ondes émis par une antenne émettrice d'un réseau (6) d'antennes micro-ondes ;
(b) recevoir les signaux micro-ondes après diffusion dans la partie corporelle au niveau de multiples antennes réceptrices (16) du réseau (6) d'antennes micro-ondes afin d'obtenir un ensemble de données ;
(c) déplacer les positions des antennes (16) par rapport à la partie corporelle de sorte qu'elles soient situées à des positions qui étaient précédemment inoccupées et répéter les étapes (a) et (b) ;
(d) concaténer les ensembles de données obtenus et générer une sortie indiquant la structure interne de la partie corporelle à partir des ensembles de données concaténés ; **caractérisé en ce que** :
l'espacement physique entre les antennes (16) est supérieur à la moitié de la longueur d'onde du signal micro-ondes et un espacement efficace entre les antennes (16), défini comme étant l'espacement entre des positions occupées sur toutes les configurations et obtenu en déplaçant les antennes (16) vers des positions précédemment inoccupées, est inférieur à la moitié de la longueur d'onde du signal micro-ondes ; et **en ce que**
les antennes (16) sont positionnées dans une formation à partir d'une tessellation normale d'un polygone qui est incliné par rapport à une direction de déplacement des antennes (16).

10. Procédé d'imagerie médicale selon la revendication 9, dans lequel les antennes (16) sont déplacées à l'aide d'un actionneur (8) relié au réseau (6) d'antennes micro-ondes.

11. Procédé d'imagerie médicale selon la revendication 10, dans lequel l'actionneur (8) est conçu pour faire tourner le réseau (6) d'antennes micro-ondes par rapport à la partie corporelle.
